# FASCICULE DE BREVET EUROPEEN

(11) **EP 2 376 530 B1**
(45) Date de publication et mention de la délivrance du brevet: **04.05.2016**
(21) Numéro de dépôt: 10704582.5
(22) Date de dépôt: 07.01.2010
(51) Int. Cl.: C07K 14/47, C07K 5/08, A61K 38/43, A61K 8/64

(54) **NOUVEAUX PEPTIDES ANTI-AGE ET COMPOSITION COSMETIQUE ET/OU PHARMACEUTIQUE LES CONTENANT**
NEUES ANTI-AGE-PEPTIDE UND KOSMETISCHE UND/ODER PHARMAZEUTISCHE ZUSAMMENSETZUNG DAMIT
NOVEL ANTI-AGEING PEPTIDES AND COSMETIC AND/OR PHARMACEUTICAL COMPOSITION CONTAINING SAME

(30) Priorité: 09.01.2009 FR 0900069
(43) Date de publication de la demande: 19.10.2011
(73) Titulaire: ISP Investments Inc., Wilmington, DE 19805 (US)
(72) Inventeur: DAL FARRA, Claude, Kerhonkson NY 12446 (US); DOMLOGE, Nouha, F-06560 Valbonne (FR); BOTTO, Jean-Marie, F-06560 Valbonne (FR); IMBERT, Isabelle, F-75010 Paris (FR)
(74) Mandataire: Macquet, Christophe
(86) Numéro de dépôt international: PCT/FR2010/000007
(87) Numéro de publication internationale: WO 2010/079285

(56) Documents cités:
- EP-A- 1 864 997
- WO-A-99/57137
- HIRAYAMA JUN ET AL: "Structural and functional features of transcription factors controlling the circadian clock" CURRENT OPINION IN GENETICS & DEVELOPMENT, vol. 15, no. 5, octobre 2005 (2005-10), pages 548-556, XP002545453 ISSN: 0959-437X
- KONDRATOV ROMAN V: "A role of the circadian system and circadian proteins in aging" AGEING RESEARCH REVIEWS, vol. 6, no. 1, mai 2007 (2007-05), pages 12-27, XP002545454 ISSN: 1568-1637
- TANIOKA MIKI ET AL: "Molecular Clocks in Mouse Skin" JOURNAL OF INVESTIGATIVE DERMATOLOGY, [Online] vol. 129, no. 5, 27 novembre 2008 (2008-11-27), pages 1225-1231, XP002545455 ISSN: 0022-202X

### SEQUENCE LISTING

<110> ISP Investments inc
<120> NOUVEAUX PEPTIDES ANTI-AGE ET COMPOSITION COSMETIQUE ET/OU PHARMACEUTIQUE LES CONTENANT
<130> Bv PCT 09-117
<150> FR0900069
   <151> 2009-01-09
<160> 4
<170> PatentIn version 3.3
<210> 1
   <211> 4
   <212> PRT
   <213> Artificial
<220>
   <223> Synthetic peptide
<220>
   <221> MOD_RES
   <222> (4)..(4)
   <223> AMIDATION
<400> 1
   Ser Pro Leu Gln
   1
<210> 2
   <211> 5
   <212> PRT
   <213> Artificial
<220>
   <223> Synthetic peptide
<400> 2
   Thr Ser Pro Leu Gln
   1 5
<210> 3
   <211> 6
   <212> PRT
   <213> Artificial
<220>
   <223> Synthetic peptide
<220>
   <221> MOD_RES
   <222> (6)..(6)
   <223> AMIDATION
<400> 3
   Ser Ser Pro Leu Gln Leu
   1 5
<210> 4
   <211> 7
   <212> PRT
   <213> Artificial
<220>
   <223> synthetic peptide
<220>
   <221> MOD_RES
   <222> (7)..(7)
   <223> AMIDATION
<400> 4
   Ser Ser Pro Leu Gln Ala Asn
   1 5

## Description

La présente invention se rapporte à des composés peptidiques de formule générale R₁ - X₁ - X₂ - Ser - Pro - Leu - Gln - X₃ - X₄ - R₂ ainsi que leurs utilisations en cosmétique et/ou pharmaceutique afin de prévenir et corriger les effets du vieillissement et les agressions UV de la peau et des phanères.

La fonction première de l'épiderme, est de constituer une barrière entre l'environnement extérieur et le milieu intérieur. C'est la couche la plus externe de l'épiderme, *le stratum corneum,* qui assure cette fonction. Il est composé de kératinocytes au stade ultime de leur différenciation, les cornéocytes, scellés les uns aux autre par un épais ciment intercellulaire, à la fois souple et imperméable. Cette barrière physique qu'est la peau permet au corps humain de se protéger de nombreux types d'agressions. Ces agressions peuvent avoir diverses origines intrinsèques, telles que le vieillissement chronologique ou encore des modifications biochimiques qui ont lieu lors d'états de fatigue, de stress, de changements hormonaux comme lors de la grossesse.... D'autres agressions elles, ont une origine extrinsèque telle que la pollution, le soleil, les maladies....En réponse à ces agressions, l'aspect de la peau est modifié et l'on voit apparaître alors des rides et ridules, des tâches d'hyper ou d'hypo-pigmentation, une sècheresse voire une déshydratation de la peau, un amincissement de l'épiderme, une élastose, des imperfections, des tâches de vieillesse.... Ces modifications sont dues à l'altération des fonctions de renouvellement cellulaire, de cohésion cellulaire, de synthèse de collagène, d'élastine et d'autres protéines et conduisent finalement à une diminution des qualités de barrière protectrice de la peau et à un aspect peu esthétique de celle-ci. Tous ces changements affectent non seulement la peau, mais également les phanères tel que les ongles et cheveux. Par exemple, les cheveux deviennent ternes, ou grisonnant, ou encore tombent de manière précoce...

Afin de remédier à ces inconvénients, de nombreuses compositions cosmétiques ont été proposées ces dernières années. Ces compositions incluaient l'utilisation, seul ou en combinaison, d'agents hydratants, apaisants, anti-inflammatoires, anti-radicaux libres, anti-séborrhéiques, cicatrisants, éclaircissants, kératolytiques... Cependant, aucune de ces compositions ne permet à l'heure actuelle, de prévenir ou de corriger les effets du vieillissement d'origine intrinsèque ou extrinsèque de la peau.

De manière surprenante, la Demanderesse a découvert que des composés peptidiques de formule générale suivante R₁ - X₁ - X₂ - Ser - Pro - Leu - Gin - X₃ - X₄ - R₂, avaient un effet anti-vieillissement sur la peau par une action globale sur divers symptômes caractéristiques de ce dernier. Aucun document de l'art antérieur ne décrit de tels composés peptidiques afin d'obtenir un effet anti-vieillissement. En outre, ces composés peptidiques sont caractérisés par le fait qu'ils sont des agents activateurs du gène Clock impliqués dans la régulation du cycle circadien. Par conséquent, la présente invention a pour objet des composés peptidiques activateurs de Clock (Circadian Locomotor Output Cycles Kaput) ainsi que leur utilisation dans des compositions cosmétiques et pharmaceutiques afin de prévenir ou corriger les signes dus au vieillissement et aux agressions UV de la peau et des phanères.

L'horloge circadienne est contrôlée par une boucle négative de régulation impliquant un ensemble de gènes, notamment les gènes Per-1 (Period), Clock et BMAL-1 (Brain and Muscle ARNt-like Protein).

Il a été démontré que le vieillissement s'accompagne d'une modification des rythmes biologiques, avec une diminution de l'amplitude et une tendance à l'avance de phase (Weinert D., Chronobiol. Int. 2000 ; 17 :261-83). En outre, il a été montré que l'horloge circadienne était altérée par ce même vieillissement.

Jusqu'à présent, dans l'art antérieur, de nombreuses applications ont déjà été proposées quant à l'utilisation des nucléotides et/ou de protéines issues des gènes Clock, Per-1 ou BMAL-1. On peut citer par exemple le brevet US 6 291 429 qui décrit l'utilisation du produit du gène Clock pour la resynchronisation du cycle du sommeil ou de processus physiologiques ou endocriniens, la resynchronisation du corps après un décalage horaire. On connaît également le document WO9957137 qui décrit des compositions comportant une protéine BMAL 1 isolée et une protéine CLOCK isolée, et comportant également la protéine PER isolée et éventuellement la protéine TIM isolée, et se rapporte aussi à des analyses dépendant de BMAL 1/CLOCK en vue de l'identification de modulateurs de l'expression du gène *period* et en outre à des méthodes permettant de diagnostiquer des troubles liés à une anomalie du rythme circadien. Cependant, aucun document de l'art antérieur ne décrit l'utilisation de peptides spécifiques activateurs de Clock pour prévenir ou corriger les signes du vieillissement ou encore les conséquences d'agressions UV.

La présente invention a pour premier objet un composé peptidique de formule (I) suivante :

R₁ - X₁ - X₂ - Ser - Pro - Leu - Gin - X₃ - X₄ - R₂

Dans laquelle,
X₁ représente une cystéine, une méthionine ou est égal à zéro,
X₂ représente une sérine, une thréonine, ou est égal à zéro,
X₃ représente une alanine, une glycine, une isoleucine, une leucine, une proline, une valine ou est égal à zéro,
X₄ représente une asparagine, une glutamine, ou est égal à zéro.

R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, libre ou substituée par un groupement protecteur pouvant être choisi parmi une chaîne alkyle de C₁ à C₂₀, ou un groupement NH2, NHY ou NYY avec Y représentant une chaîne alkyle de C₁ à C₄,
ladite séquence de formule générale (I) étant constituée de 4 à 8 résidus d'acides aminés.

La présente invention a pour second objet une composition cosmétique ou dermo-pharmaceutique comprenant des composés peptidiques de formule générale (I).

La présenté invention a pour troisième objet un composé peptidique de formule générale (I) comme principe actif activateur de Clock.

La présente invention a pour quatrième objet l'utilisation d'une composition cosmétique ou dermo- pharmaceutique comprenant des composés peptidiques de formule générale (I) pour prévenir ou traiter les signes cutanés du vieillissement.

La présente invention a pour cinquième objet une composition cosmétique ou dermopharmaceutique comprenant des composés peptidiques de formule générale (1) pour protéger la peau contre les agressions dues aux rayonnements UV.

Enfin, la présente invention a pour sixième objet une composition cosmétique ou dermo-pharmaceutique appliquée topiquement sur la peau ou les phanères à traiter avant le coucher de façon à respecter le rythme circadien de la peau pour obtenir un effet rajeunissant sur la peau..

Le premier objet de l'invention se rapporte à un composé peptidique de formule générale (I) suivante :

R₁ - X₁ - X₂ - Ser - Pro - Leu - Gin - X3 - X₄ - R₂

Dans laquelle,
X₁ représente une cystéine, une méthionine ou est égal à zéro,
X₂ représente une sérine, une thréonine, ou est égal à zéro,
X₃ représente une alanine, une glycine, une isoleucine, une leucine, une proline, une valine ou est égal à zéro,
X₄ représente une asparagine, une glutamine, ou est égal à zéro.

R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, libre ou substituée par un groupement protecteur pouvant être choisi parmi une chaîne alkyle de C₁ à C₂₀, ou un groupement NH2, NHY ou NYY avec Y représentant une chaîne alkyle de C₁ à C₄,
ladite séquence de formule générale (I) étant constituée de 4 à 8 résidus d'acides aminés.

De façon à améliorer la résistance à la dégradation, il peut être nécessaire d'utiliser une forme protégée du peptide selon l'invention. La forme de protection doit évidemment être une forme biologiquement compatible et doit être compatible avec une utilisation dans le domaine des cosmétiques ou de la pharmacie. De manière préférentielle, le composé peptidique possède au moins un groupement fonctionnel protégé par un groupement protecteur, ce groupement protecteur étant soit une acétylation de l'extrémité amino-terminale, soit une amidation ou une estérification de l'extrémité carboxy-terminale, soit les deux. De préférence, on utilise une protection basée sur l'amidation de la fonction hydroxyle de l'extrémité carboxy-terminale par un groupement NYY avec Y représentant une chaîne alkyle de C₁ à C₄, ou l'estérification par un groupement alkyle.

Préférentiellement, le composé peptidique présente l'une des séquences suivantes :
(SEQ ID n°1) Ser - Pro - Leu - Gln - NH₂
(SEQ ID n°2) Thr - Ser - Pro - Leu - Gln
(SEQ ID n°3) Ser - Ser - Pro - Leu - Gln -Leu - NH₂
(SEQ ID n°4) Ser - Ser - Pro - Leu - Gln - Ala - Asn - NH₂

Dans un mode de réalisation particulier, la séquence du composé peptidique est la séquence SEQ ID n°1, c'est-à-dire Ser - Pro - Leu - Gln - NH₂.

L'invention concerne aussi des formes homologues de ces séquences. Le terme « homologue » désigne, selon l'invention, toute séquence peptidique identique à au moins 50%, ou de préférence au moins 80%, et encore plus préférentiellement à au moins 90% de ladite séquence peptidique, choisie parmi les séquences SEQ ID n° 1 à SEQ ID n° 4. Par « séquence peptidique identique à au moins X% », on entend désigner un pourcentage d'identité entre les résidus d'acides aminés des deux séquences à comparer, obtenu après l'alignement optimal des deux séquences. L'alignement optimal est obtenu à l'aide d'algorithmes d'homologies locales tels que ceux utilisés par les logiciels informatiques BLAST P ou T BLAST N disponibles sur le site NCBI.

Le terme « homologue » peut également désigner un peptide qui diffère de la séquence d'un peptide de séquence SEQ ID n° 1 à SEQ ID n° 4 par la substitution d'acides aminés chimiquement équivalents, c'est-à-dire par la substitution d'un résidu par un autre possédant les mêmes caractéristiques. Ainsi, les substitutions classiques se font entre Ala, Val, Leu et Ile ; entre Ser et Thr ; entre les résidus acides Asp et Glu ; entre Asn et Gln; et entre les résidus basiques Lys et Arg ; ou entre les résidus aromatiques Phe et Tyr.

Le terme « peptide » désigne un enchaînement de deux ou plusieurs acides aminés liés entre eux par des liaisons peptidiques ou par des liaisons peptidiques modifiées.

Par « peptide », il faut entendre le peptide naturel ou synthétique de l'invention tel que décrit ci-dessus, ou au moins l'un de ses fragments, qu'il soit obtenu par protéolyse ou de manière synthétique, ou encore tout peptide naturel ou synthétique dont la séquence est totalement ou partiellement constituée par la séquence du peptide précédemment décrit.

Le peptide de formule générale (I) selon l'invention peut être obtenu soit par synthèse chimique classique (en phase solide ou en phase homogène liquide), soit par synthèse enzymatique (Kullman et al., J. Biol. Chem. 1980, 225, 8234), à partir d'acides aminés constitutifs ou de leurs dérivés.

Le peptide selon l'invention peut être d'origine naturelle ou synthétique. Préférentiellement selon l'invention, le peptide est obtenu par synthèse chimique.

Enfin, le principe actif peut être un peptide unique, un mélange de peptides ou de dérivés peptidiques et/ou constitués de dérivés d'acides aminés.

Le composé peptidique selon l'invention peut être utilisé à titre de médicament.

Le second objet de la présente invention se rapporte à des compositions cosmétiques comprenant ledit composé peptidique de formule générale (I). De manière préférée, les compositions selon l'invention se présentent sous une forme adaptée à l'application par voie topique comprenant un milieu cosmétiquement ou dermatologiquement acceptable. Par « cosmétiquement ou dermatologiquement acceptable », on entend des milieux qui conviennent à une utilisation en contact avec la peau ou les phanères humains, sans risque de toxicité, d'incompatibilité, d'instabilité, de réponse allergique et autres. Préférentiellement, ledit composé peptidique est présent dans la composition à une concentration comprise entre 0,0005 et 500 ppm environ, et préférentiellement à une concentration comprise entre 0,01 et 5 ppm. Dans les compositions selon l'invention, le composé peptidique est préalablement solubilisé dans un ou plusieurs solvants cosmétiquement ou dermatologiquement acceptables, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux, le principe actif selon l'invention est préalablement solubilisé dans un vecteur cosmétique ou pharmaceutique comme les liposomes ou adsorbé sur des polymères organiques poudreux, des supports minéraux comme les talcs et bentonites, et plus généralement solubilisé dans, ou fixé sur, tout vecteur physiologiquement acceptable.

Les compositions destinées à être appliquées sur la peau peuvent se présenter sous forme de solution aqueuse ou hydroalcoolique, d'émulsion eau dans huile ou huile dans eau, de microémulsion, de gel aqueux ou anhydre, de sérum, ou encore de dispersion de vésicules, de patch, de crème, de spray, d'onguent, de pommade, de lotions, de colloïde, de solution, de suspension ou autres. Les compositions peuvent aussi être appliquées sur les phanères sous forme de shampoing, de teinture ou de mascara à appliquer au pinceau ou au peigne, en particulier sur les cils, les sourcils ou les cheveux, ou encore de soins pour les ongles tels que les vernis.

Dans un mode de réalisation particulier, la composition selon l'invention contient en outre, au moins un autre principe actif favorisant l'action dudit principe actif peptidique. On peut citer, de manière non limitative, les classes d'ingrédients suivantes : d'autres agents actifs peptidiques, des extraits de végétaux, des agents cicatrisants, anti-âge, anti-rides, apaisants, anti-radicalaire, anti-UV, des agents stimulant la synthèse de macromolécules dermiques ou le métabolisme énergétique, des agents hydratants, anti-bactériens, anti-fongiques, anti-inflammatoires, anesthésiques, des agents modulants la différenciation, la pigmentation ou la dépigmentation cutanée, des agents stimulants la pousse des ongles ou des cheveux.... Préférentiellement, on utilisera un agent anti-radicalaire ou antioxydant, ou un agent stimulant la synthèse de macromolécules dermiques, ou encore un agent stimulant le métabolisme énergétique.

En outre, des additifs tels que des agents épaississants, émulsifiants, humectants, émollients, des parfums, des antioxydants, des agents filmogènes, chélatants, séquestrants, conditionnants....peuvent être ajoutés à la composition.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Un troisième objet de la présente invention se rapporte à l'utilisation du composé peptidique tel que défini précédemment en tant que principe actif activateur de Clock. On entend par composé peptidique « activateur de Clock», tout peptide ou dérivé biologiquement actif capable d'augmenter l'activité de Clock, soit par augmentation de la synthèse protéique de Clock (par modulation directe ou indirecte de l'expression génique de Clock) soit par d'autres processus biologiques tels que la stabilisation de la protéine Clock ou encore la stabilisation des transcrits d'ARN messager.

Un quatrième objet de la présente invention se rapporte à l'utilisation d'une composition comprenant ledit composé peptidique pour prévenir ou traiter les signes cutanés du vieillissement. Les « signes cutanés du vieillissement » incluent, mais ne se limitent pas à, toutes les manifestations visibles sur la peau causés par le vieillissement. On entend notamment, les rides, ridules, crevasses, pores élargis, imperfections, perte de fermeté, décoloration, tâches de vieillesse, kératoses, perte de collagène, et autres changements du derme et de l'épiderme....Par « signes cutanés du vieillissement », on entend également toutes modifications de l'aspect extérieur de la peau et des phanères dues au vieillissement comme, par exemple, les rugosités superficielles de la couche cornée, mais également toute modification interne de la peau qui ne se traduit pas systématiquement par un aspect extérieur modifié comme, par exemple, l'amincissement du derme ou toute autre dégradation interne de la peau.

En outre, la présente invention se rapporte à l'utilisation d'une composition selon ladite invention pour la protection de la peau contre les agressions dues aux rayonnements UV.

Un autre objet de l'invention concerne l'utilisation d'une quantité efficace de principe actif peptidique selon l'invention, pour la préparation d'une composition pharmaceutique destinée à prévenir ou à lutter contre les pathologies liées aux processus d'oxydation.

Un dernier objet de la présente invention se rapporte à ce que l'on applique topiquement sur la peau ou les phanères à traiter une composition contenant une quantité efficace de principe actif peptidique pour prévenir ou traiter les signes cutanés du vieillissement ou protéger la peau contre les agressions dues aux rayonnements UV. La composition est appliquée avant le coucher de façon à respecter le rythme circadien de la peau pour obtenir un effet rajeunissant sur la peau. En effet, durant la nuit, la peau privilégie des fonctions de renouvellement ainsi que des processus métaboliques de synthèse. Par conséquent, l'application de la composition telle que revendiquée, en respectant le rythme biologique de la peau permet d'obtenir un effet rajeunissant, stimulant le renouvellement cellulaire, et régénérant.

Les exemples suivants décrivent et démontrent l'efficacité de composés peptidiques tels que décrits selon l'invention. La formulation cosmétique citée est représentative de l'invention mais est donnée uniquement à titre d'illustration et ne doit pas être interprétée comme une limitation de la présente invention.

### Exemple 1 : Mise en évidence de l'effet activateur du peptide SEQ ID n°1 sur l'expression de la protéine Clock sur des fibroblastes en culture.

Une étude de l'effet activateur du peptide SEQ ID n°1, a été réalisée en évaluant l'expression de la protéine Clock en western blot sur des fibroblastes en culture et en immunohistochimie par marquage de la protéine Clock par immunomarquage. La technique de western blotting est une méthode semi quantitative qui permet d'apprécier le taux de protéine Clock dans les cellules dermiques.

### Protocole

### a) Etude en western blot

Des fibroblastes en cultures sont cultivés dans des boîtes de diamètre 100 mm à 37°C dans une atmosphère humidifiée contenant 5% de C02 pendant 16 h en présence ou non du peptide SEQ ID n°1, dilué 1% (à partir d'une solution 10⁻⁴M). Les cellules sont rincées puis détachées du support à l'aide d'un tampon d'extraction (20mM TRIS, 150mM NaCl, 10mM EDTA, 0.2% Triton X10) en présence d'un cocktail d'inhibiteurs de protéases (Sigma). Les protéines ainsi extraites, sont centrifugées à 4°C à 10000 rpm, pendant avant d'être dosées par le kit de dosage des protéines BCA (Pierce). Les lysats cellulaires sont mélangés à un tampon de dénaturation et soumis à une électrophorèse SDS-PAGE. Le gel utilisé est un Nupage 4-12% (invitrogen). Les protéines sont enfin transférées sur une membrane de nitrocellulose (Pal corporation). Les membranes sont saturées dans du PBS-lait 5%, 0.1% tween 20 2 heures à température ambiante, puis incubées à 4°C toute la nuit avec un anticorps primaire anti-clock au 1/1000^{ème} (ABcam) suivie d'une incubation avec un anticorps secondaire anti-lapin Iggy-peroxydase dilué au 1/5000^{ème}. La révélation est effectuée par un substrat chimioluminescent. L'évaluation quantitative des protéines présentes dans les cellules est effectuée grâce au logiciel chemiimager (Alpha innotech Corporation USA). La quantité des protéines est exprimée en pourcentage d'intensité lumineuse comparée à la condition contrôle qui n'ont pas eu le traitement.

### b) Etude en Immunohistochimie

Des fibroblastes en cultures sont cultivés dans des chambres à 8 puits (labteck) à 37°C dans une atmosphère humidifiée contenant 5% de CO2 pendant 18 h en présence ou non du peptide SEQ ID n°1, dilué 1% (à partir d'une solution 10⁻⁴M). Des cellules non traitées sont cultivées en même temps et servent de contrôle. Le jour du marquage, les cellules sont rincées, fixées dans un mélange (2% glutaraldéhyde - 2% formaldéhyde) 3 minutes puis rincées. On applique l'anticorps primaire (ABcam) dilué au 1/250ème pendant 1 heure et un anticorps secondaire au 1/50^{ème} couplé à un fluorochrome pendant 1 heure. Les cellules sont montées entre lame et lamelle dans un milieu de montage adéquate et observées au microscope à épifluorescence.

### Résultats :

Les résultats obtenus en western blot montrent que le traitement par le peptide SEQ ID n°1, dilué 1% augmente la quantité de protéine Clock présente dans les fibroblastes en culture. Les résultats sont résumés dans le tableau ci-dessous.

| Intensité (%) | Expérience 1 |
|---|---|
| Contrôle non traités | 100% |
| 1% traitement | 179% |

Ces résultats sont confirmés par le marquage de la protéine Clock sur les fibroblastes. En effet, on montre une augmentation de l'expression de la protéine Clock sur les fibroblastes traités.

### Conclusion :

L'administration du peptide SEQ ID n°1 permet d'augmenter quantité de protéine Clock sur des cellules dermiques en culture.

### Exemple 2 : Mise en évidence de l'effet activateur du peptide SEQ ID n°1 sur l'expression de la protéine Clock et Per sur des biopsies de peaux.

Une étude de l'effet activateur du peptide SEQ ID n°1, a été réalisée en évaluant l'expression des protéines Clock et Per-1 sur des biopsies de peaux *ex vivo.*

### Protocole

Des études ex vivo par immunomarquage ont permis de mettre en évidence l'expression des protéines Clock et Per-1, sur des échantillons de peau. Des échantillons de peau humaine, sont mis en culture à l'interface air/liquide. Le peptide SEQ ID n°1 dilué à 1% (à partir d'une solution 10⁻⁴M) est appliqué de façon topique sur ces échantillons pendant 48 h à raison de 2 fois par 24 h.

Des échantillons de peaux non traitées par le peptide SEQ ID n°1 servent de contrôle. Les échantillons de peaux sont fixés au formaldéhyde 10% et inclus dans la paraffine. Des coupes de 3µm sont réalisées au microtome. L'immunomarquage est effectué après un déparaffinage et un démasquage des sites antigéniques.

L'immunomarquage s'effectue pour la protéine Clock, par un anticorps (ABcam) dilué au 1/250ème et un anticorps secondaire au 1/50^{ème} couplé à un fluorochrome.

L'immunomarquage s'effectue pour la protéine Per-1, par un anticorps polyclonal (Cosmobio) dilué au 1/100^{ème} et un anticorps secondaire au 1/50^{ème} couplé à un fluorochrome.

Les lames sont alors montées dans un milieu adéquat et observé au microscope à épifluorescence (Nikon Eclipse E600).

### Résultats :

Les résultats obtenus montrent que les peaux traitées avec le peptide SEQ ID n° 1 expriment une quantité de protéine Clock nettement supérieure aux peaux non traitées.

De même on montre que les peaux traitées avec le peptide SEQ ID n°1 expriment une quantité de protéine Per-1 supérieure aux peaux non traitées.

L'évaluation semi-quantitative du marquage est effectuée par observation microscopique et résumé dans le tableau ci-dessous.

| | Non traité | Traité par peptide SEQ ID n°1 dilué à 1% |
|---|---|---|
| Marquage protéine Clock | +/- | ++ |
| Marquage protéine PER-1 | + | + |

### Conclusion :

Le peptide SEQ ID n°1 permet, d'augmenter la quantité des protéines Clock et Per-1 sur des biopsies de peaux.

### Exemple 3 : Mise en évidence de l'effet activateur du peptide SEQ ID n°1 sur l'expression de la protéine Clock et Per-1, sur des biopsies de peau au cours du vieillissement

Une étude de l'effet activateur du peptide SEQ ID n°1, a été réalisée en évaluant l'expression des protéines Clock et Per-1 sur des modèles de peaux, vieillis artificiellement en culture. L'analyse s'est effectuée après 62 h et 134 h de culture.

### Protocole

Des études ex vivo par immunomarquage ont permis de mettre en évidence l'expression des protéines Clock et Per-1, sur des échantillons de peaux vieillis en culture. Des échantillons de peau humaine, sont mis en culture à l'interface air/liquide, pendant 62 h et 134 h. Le peptide SEQ ID n°1 dilué à 1% (à partir d'une solution 10⁻⁴M).est appliqué de façon topique sur ces échantillons à raison de 2 fois par jour pendant le temps de l'expérience.

Des échantillons de peau non traitées par le peptide SEQ ID n°1 servent de contrôle. Les échantillons de peaux sont fixés au formaldéhyde 10% et inclus dans la paraffine. Des coupes de 3µm sont réalisées au microtome. L'immunomarquage est effectué après un déparaffinage et un démasquage des sites antigéniques. L'immunomarquage s'effectue pour la protéine Clock, par un anticorps (ABcam) dilué au 1/250ème et un anticorps secondaire au 1/50^{ème} couplé à un fluorochrome.

L'immunomarquage s'effectue pour la protéine Per-1, par un anticorps polyclonal (Cosmobio) dilué au 1/100^{ème} et un anticorps secondaire au 1/50^{ème} couplé à un fluorochrome.

Les lames sont alors montées dans un milieu adéquat et observées au microscope à épifluorescence (Nikon Eclipse E600).

### Résultats :

Les résultats obtenus montrent que le marquage de la protéine Clock (essentiellement épidermique) est augmenté après 62 h de culture, par rapport aux lames témoins non traitées. De même, l'expression de la protéine Per-1 est augmentée après 62 h de traitement. Ce marquage perdure dans le temps puisqu'au bout de 134 h, le marquage de la protéine Clock est fortement augmenté par rapport aux peaux témoins non traitées. Le tableau ci-dessous donne une évaluation visuelle du marquage.

| | | Non traité | Traité par peptide SEQ ID n°1 dilué à 1% |
|---|---|---|---|
| Marquage Clock | 62 h | + | ++ |
| | 134 h | + | ++ |
| Marquage Per-1 | 62 h | +/- | ++ |
| | 134 h | +/- | + |

### Conclusion :

Le peptide SEQ ID n°1 permet, d'augmenter la quantité des protéines Clock et Per-1 sur des biopsies de peau traitées et vieillies artificiellement en culture.

### Exemple 4 : Mise en évidence de l'effet protecteur du peptide SEQ ID n°1 sur des biopsies de peau au cours du vieillissement

Parallèlement une étude de l'effet protecteur du peptide SEQ ID n°1, a été réalisée en évaluant en immunohistologie au moyen d'une coloration de coupes de peau les dommages causés un vieillissement de 62 h et 134 h.

### Protocole

Des échantillons de peau humaine, sont mis en culture à l'interface air/liquide. Le peptide SEQ ID n°1 dilué à 1% (à partir d'une solution 10⁻⁴M).est appliqué de façon topique sur ces échantillons pendant 62 h et 134 h. L'application est renouvelée 2 fois par jour pendant le temps de l'expérience. Des échantillons de peau non traitées servent de contrôle pendant le temps de l'expérience. Les échantillons de peaux sont fixés au formaldéhyde 4% et inclus dans la paraffine. Des coupes de 3µm sont réalisées au microtome. Une coloration des tissus est faite par l'hématoxyline (qui colore les noyaux en bleu-noir) et l'éosine qui est spécifique de la coloration des cytoplasmes. La structure morphologique des biopsies est alors appréciée au microscope à transmission.

### Résultats :

Les résultats obtenus montrent que les peaux non traitées avec le peptide SEQ ID n° 1 sont endommagées, les cellules sont vacuolées, les cellules paraissent endommagées avec des cellules dont les noyaux sont très condensés (signes apoptotiques). Au contraire les peaux traitées avec le peptide SEQ ID n°1 ont une structure morphologique bien conservée, les dégâts dus au vieillissement sont moins visibles. Pour les 2 expériences 62 et 134 h le peptide SEQ ID n°1 protège les biopsies de peaux du vieillissement.

### Conclusion :

Le peptide SEQ ID n°1 permet de mettre en évidence un effet protecteur sur la morphologie structurale des biopsies de peaux soumises à un vieillissement artificiel en culture.

### Exemple 7: Préparation d'une composition

**Crème protection solaire:**

| Noms commerciaux | Noms INCI | % massique |
|---|---|---|
| PHASE A | | |
| Eau déminéralisée | Aqua (Water) | qsp |
| Pemulen TR1 | Acrylates/C10-30 Alkyl | 0,40 |
| | Acrylate Crosspolymer | |
| Glycerine | Glycerin | 3,00 |
| Nipastat Sodium | Sodium Methylparaben (and) | 0,15 |
| | Sodium Ethylparaben (and) | |
| | Sodium Butyl paraben (and) | |
| | Sodium Propylparaben (and) | |
| | Sodium Isobutylparaben | |

| PHASE B | | |
|---|---|---|
| Parsol MCX | Ethylhexyl Methoxycinnamate | 7,50 |
| Eusolex 4360 | Benzophenone-3 | 3,00 |
| Parsol 1789 | Butyl Methoxydibenzoylmethane | 2,00 |
| Myritol 318 | Caprylic/Capric Triglyceride | 4,00 |
| Emulgade SEV | Hydrogenated Palm Glycerides (and) Ceteareth-20 (and) Ceteareth-12 (and) Cetearyl Alcohol | 5,00 |
| Propylparaben | Propylparaben | 0,15 |
| Nacol 16-98 | Cetyl Alcohol | 1,00 |

| PHASE C | | |
|---|---|---|
| TEA | Triethanolamine | 0,20 |

| PHASE D | | |
|---|---|---|
| Peptide SEQ ID n°2 | | 3 ppm |
| Parfum | Parfum (Fragrance) | qsp |
| Colorant | | qsp |

Les constituants de la phase A et de la phase B sont chauffés séparément entre 70°C et 75°C. La phase B est émulsionnée dans la phase A sous agitation. La phase C est ajoutée, à 45°C, en augmentant l'agitation. La phase D est ensuite additionnée lorsque la température se situe en dessous de 40°C. Le refroidissement est poursuivi jusqu'à 25°C sous vive agitation.

## Revendications

1. Composé peptidique de formule générale suivante (I) :
R₁-X₁-X₂-Ser-Pro-Leu-Gln-X₃-X₄-R₂
Dans laquelle,
X₁ représente une cystéine, une méthionine ou est égal à zéro,
X₂ représente une sérine, une thréonine, ou est égal à zéro,
X₃ représente une alanine, une glycine, une isoleucine, une leucine, une proline, une valine ou est égal à zéro,
X₄ représente une asparagine, une glutamine, ou est égal à zéro.
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, libre ou substituée par un groupement protecteur pouvant être choisi parmi une chaîne alkyle de C₁ à C₂₀, ou un groupement NH2, NHY ou NYY avec Y représentant une chaîne alkyle de C₁ à C₄,
ladite séquence de formule générale (I) étant constituée de 4 à 8 résidus d'acides aminés.

2. Composé peptidique selon la revendication 1, **caractérisé en ce que** le peptide de formule générale (I) possède au moins un groupement fonctionnel protégé par un groupement protecteur, ce groupement protecteur étant soit une acétylation de l'extrémité amino-terminale, soit une amidation ou une estérification de l'extrémité carboxy-terminale, soit les deux.

3. Composé peptidique selon l'une des revendications 1 ou 2, **caractérisé en ce qu'**il correspond à une des formules suivantes :
(SEQ ID n°1) Ser - Pro - Leu - Gln - NH₂
(SEQ ID n°2) Thr - Ser - Pro - Leu - Gln
(SEQ ID n°3) Ser - Ser - Pro - Leu - Gln - Leu - NH₂
(SEQ ID n°4) Ser - Ser - Pro - Leu - Gln - Ala - Asn - NH₂

4. Composé peptidique selon l'une des revendications précédentes **caractérisé en ce qu'**il est utilisé à titre de médicament.

5. Composition cosmétique comprenant à titre de principe actif ledit composé peptidique selon l'une des revendications 1 à 3.

6. Composition cosmétique selon la revendication 5, **caractérisée en ce qu'**elle se présente sous une forme adaptée à l'application par voie topique comprenant un milieu cosmétiquement acceptable.

7. Composition cosmétique selon l'une des revendications 5 ou 6, **caractérisée en ce que** ledit composé peptidique est présent dans la composition à une concentration comprise entre 0,0005 et 500 ppm environ, et préférentiellement à une concentration comprise entre 0,01 et 5 ppm.

8. Composition cosmétique selon l'une quelconque des revendications 5 à 7, **caractérisée en ce que** ledit principe actif peptidique est solubilisé dans un ou plusieurs solvants, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

9. Composition cosmétique selon l'une quelconque des revendications 5 à 8, **caractérisée en ce qu'**elle contient en outre, au moins un autre principe actif favorisant l'action dudit principe actif peptidique choisi parmi un agent anti-radicalaire ou antioxydant, ou un agent stimulant la synthèse de macromolécules dermiques, ou encore un agent stimulant le métabolisme énergétique.

10. Composé peptidique tel que défini dans l'une des revendications 1 à 4 en tant que principe actif activateur de Clock.

11. Composition selon l'une quelconque des revendications 5 à 9, pour prévenir ou traiter les signes cutanés du vieillissement.

12. Composition selon l'une quelconque des revendications 5 à 9, pour protéger la peau contre les agressions dues aux rayonnements UV.

13. Composition selon l'une quelconque des revendications 5 à 9, **caractérisée en ce qu'**elle est appliquée topiquement sur la peau ou les phanères à traiter avant le coucher de façon à respecter le rythme circadien de la peau pour obtenir un effet rajeunissant sur la peau.

## Patentansprüche

1. Peptidverbindung mit der folgenden allgemeinen Formel (I) :
R₁ - X₁- X₂ - Ser - Pro - Leu - Gln - X₃ - X₄ - R₂
wobei
X₁ ein Cystein, ein Methionin darstellt oder gleich Null ist,
X₂ ein Serin, ein Threonin darstellt oder gleich Null ist,
X₃ ein Alanin, ein Glycin, ein Isoleucin, ein Leucin, ein Prolin, ein Valin darstellt oder gleich Null ist,
X₄ ein Asparagin, ein Glutamin darstellt, oder gleich Null ist,
R₁ die primäre Aminofunktion der N-terminalen Aminosäure darstellt, frei oder substituiert durch eine Schutzgruppe, die aus einer Acetylgruppe, einer Benzoylgruppe, einer Tosylgruppe oder einer Benzyloxycarbonylgruppe gewählt werden kann,
R₂ die Hydroxylgruppe der Carboxylfunktion der C-terminalen Aminosäure darstellt, frei oder substituiert durch eine Schutzgruppe, die aus einer C₁ bis C₂₀-Alkylkette oder einer NH2-, NHY- oder NYY-Gruppe gewählt werden kann, wobei Y eine C₁ bis C₄-Alkylkette darstellt, wobei die Sequenz mit der allgemeinen Formel (I) aus 4 bis 8 Aminosäureresten besteht.

2. Peptidverbindung nach Anspruch 1, **dadurch gekennzeichnet, dass** das Peptid mit der allgemeinen Formel (I) mindestens eine funktionelle Gruppe aufweist, die von einer Schutzgruppe geschützt ist, wobei diese Schutzgruppe entweder eine Acetylierung des aminoterminalen Endes ist oder eine Amidbildung oder eine Veresterung des carboxy-terminalen Endes oder beides ist.

3. Peptidverbindung nach einem der Ansprüche 1 oder 2, **dadurch gekennzeichnet, dass** sie einer der folgenden Formeln entspricht:
(SEQ ID Nr. 1) Ser - Pro - Leu - Gln - NH₂
(SEQ ID Nr. 2) Thr - Ser - Pro - Leu - Gln
(SEQ ID Nr. 3) Ser - Ser - Pro - Leu - Gln - Leu - NH₂
(SEQ ID Nr. 4) Ser - Ser - Pro - Leu - Gln - Ala - Asn - NH₂

4. Peptidverbindung nach einem der vorhergehenden Ansprüche, **dadurch gekennzeichnet, dass** sie als Arzneimittel verwendet wird.

5. Kosmetische Zusammensetzung, umfassend als Wirkstoff die Peptidverbindung nach einem der Ansprüche 1 bis 3.

6. Kosmetische Zusammensetzung nach Anspruch 5, **dadurch gekennzeichnet, dass** sie in einer Form vorliegt, die ausgelegt ist, um auf topischem Weg angewendet zu werden, umfassend ein annehmbares kosmetisches Medium.

7. Kosmetische Zusammensetzung nach einem der Ansprüche 5 oder 6, **dadurch gekennzeichnet, dass** die Peptidverbindung in der Zusammensetzung in einer Konzentration vorhanden ist, die ungefähr zwischen 0,0005 und 500 ppm liegt, und vorzugsweise in einer Konzentration, die zwischen 0,01 und 5 ppm liegt.

8. Kosmetische Zusammensetzung nach einem der Ansprüche 5 bis 7, **dadurch gekennzeichnet, dass** der Peptidwirkstoff in einem oder in mehreren Lösemitteln solubilisiert ist, wie z.B. Wasser, Glycerol, Ethanol, Propylenglykol, Butylenglykol, Dipropylenglykol, ethoxylierte oder propoxylierte Diglykole, cyclische Polyole, Vaselin, einem pflanzlichen Öl oder jeder Mischung dieser Lösemittel.

9. Kosmetische Zusammensetzung nach einem der Ansprüche 5 bis 8, **dadurch gekennzeichnet, dass** sie außerdem mindestens einen weiteren Wirkstoff umfasst, der die Wirkung des Peptidwirkstoffs begünstigt, ausgewählt aus einem antiradikalen Mittel oder Antioxidationsmittel oder einem Mittel, das die Synthese von Hautmakromolekülen stimuliert oder auch einem Mittel, das den Energiestoffwechsel stimuliert.

10. Peptidverbindung, wie in einem der Ansprüche 1 - 4 definiert, als aktivierender Wirkstoff von Clock.

11. Zusammensetzung nach einem der Ansprüche 5 bis 9, um den Hautzeichen des Alterns vorzubeugen oder sie zu behandeln.

12. Zusammensetzung nach einem der Ansprüche 5 bis 9, um die Haut vor den Aggressionen aufgrund von UV-Strahlungen zu schützen.

13. Zusammensetzung nach einem der Ansprüche 5 bis 9, **dadurch gekennzeichnet, dass** sie topisch auf die Haut oder die Hautanhangsgebilde, die behandelt werden sollen, vor dem Schlafen aufgebracht wird, um die circadiane Rhythmik der Haut zu respektieren, um eine verjüngende Wirkung auf die Haut zu erzielen.

## Claims

1. Peptide compound having the following general formula (I):
R₁-X₁-X₂-Ser-Pro-Leu-Gln-X₃-X₄-R₂
Wherein,
X₁ represents a cysteine, a methionine or is equal to zero,
X₂ represents a serine, a threonine, or is equal to zero,
X₃ represents an alanine, a glycine, an isoleucine, a leucine, a proline, a valine or is equal to zero,
X₄ represents an asparagine, a glutamine, or is equal to zero,
R₁ represents the primary amine function of the N-terminal amino acid, free or substituted by a protecting group that may be chosen from among an acetyl group, a benzoyl group, a tosyl group or a benzyloxycarbonyl group, R₂ represents the hydroxyl group of the carboxyl function of the C-terminal amino acid, free or substituted by a protecting group that may be chosen from among a C₁ to C₂₀ alkyl chain, or an NH2, NHY or NYY group where Y represents a C₁ to C₄ alkyl chain,
said sequence having the general formula (I) consisting of 4 to 8 amino acid residues.

2. Peptide compound according to claim 1, **characterised in that** the peptide having general formula (I) has at least one functional group protected by a protecting group, this protecting group being either an acylation or an acetylation of the amino-terminus, or an amidation or an esterification of the carboxy-terminus, or both.

3. Peptide compound according to any one of claims 1 or 2, **characterised in that** it corresponds to one of the following formulae:
(SEQ ID No. 1) Ser-Pro-Leu-Gln-NH₂
(SEQ ID No. 2) Thr-Ser-Pro-Leu-Gln
(SEQ ID No. 3) Ser-Ser-Pro-Leu-Gln-Leu-NH₂
(SEQ ID No. 4) Ser-Ser-Pro-Leu-Gln-Ala-Asn-NH₂

4. Peptide compound according to any one of the preceding claims **characterised in that** it is used as a medicine.

5. Cosmetic composition comprising, as an active ingredient, said peptide compound according to any one of claims 1 to 3.

6. Cosmetic composition according to claim 5, **characterised in that** it is presented in a form suitable for topical application comprising a cosmetically acceptable medium.

7. Cosmetic composition according to any one of claims 5 or 6, **characterised in that** said peptide compound is present in the composition at a concentration between approximately 0.0005 and 500 ppm, and preferably at a concentration between 0.01 and 5 ppm.

8. Cosmetic composition according to any one of claims 5 to 7, **characterised in that** said peptide active ingredient is solubilised in one or a plurality of solvents, such as water, glycerol, ethanol, propylene glycol, butylene glycol, dipropylene glycol, ethoxylated or propoxylated diglycols, cyclic polyols, petroleum jelly, a vegetable oil or any mixture of these solvents.

9. Cosmetic composition according to any one of claims 5 to 8, **characterised in that** it further contains at least one further active ingredient promoting the action of said peptide active ingredient chosen from among an anti-radical or anti-oxidant agent or an agent stimulating the synthesis of dermal macromolecules, or else an agent stimulating the energy metabolism.

10. Peptide compound as defined in any one of claims 1 to 4 as a clock activation active ingredient.

11. Composition according to any one of claims 5 to 9, for preventing or treating the skin signs of ageing.

12. Composition according to any one of claims 5 to 9, for protecting the skin against damage caused by UV radiation.

13. Composition according to any one of claims 5 to 9, **characterised in that** it is applied to the skin or skin appendages to be treated before going to sleep so as to respect the circadian rhythm of the skin in order to obtain a rejuvenating effect on the skin.
